(19) Europäisches Patentamt
European Patent Office
Office européen des brevets

(11) **EP 3 231 897 B1**

(12) **EUROPEAN PATENT SPECIFICATION**

(45) Date of publication and mention
of the grant of the patent:
**25.08.2021 Bulletin 2021/34**

(51) Int Cl.:
**C23F 11/08** (2006.01)   **C12N 1/20** (2006.01)
**C12R 1/63** (2006.01)

(21) Application number: **14907975.8**

(22) Date of filing: **11.12.2014**

(86) International application number:
**PCT/CN2014/093639**

(87) International publication number:
**WO 2016/090618 (16.06.2016 Gazette 2016/24)**

(54) **BIOLOGICAL METAL CORROSION INHIBITOR AND USE THEREOF**

BIOLOGISCHER METALLKORROSIONSHEMMER UND VERWENDUNG DAVON

INHIBITEUR BIOLOGIQUE DE CORROSION MÉTALLIQUE ET SON UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**18.10.2017 Bulletin 2017/42**

(73) Proprietor: **Ningbo Institute of Materials Technology and Engineering, Chinese Academy of Sciences
Ningbo, Zhejiang 315201 (CN)**

(72) Inventors:
• **SONG, Zhenlun**
  **Ningbo**
  **Zhejiang 315201 (CN)**
• **MASOUMEH, Moradi Haghighi**
  **Ningbo**
  **Zhejiang 315201 (CN)**
• **XIAO, Tao**
  **Ningbo**
  **Zhejiang 315201 (CN)**

(74) Representative: **Ter Meer Steinmeister & Partner
Patentanwälte mbB
Nymphenburger Straße 4
80335 München (DE)**

(56) References cited:
**WO-A1-97/49289**      **WO-A2-02/40746**
**WO-A2-2014/087139**   **CN-A- 1 422 984**

CN-A- 104 480 472

• E. CHALKIADAKIS ET AL: "Partial characterization of an exopolysaccharide secreted by a marine bacterium, Vibrio neocaledonicus sp. nov., from New Caledonia", JOURNAL OF APPLIED MICROBIOLOGY., vol. 114, no. 6, 12 April 2013 (2013-04-12), pages 1702-1712, XP055474765, GB ISSN: 1364-5072, DOI: 10.1111/jam.12184
• RONGJUN ZUO: "Biofilms: strategies for metal corrosion inhibition employing microorganisms", APPLIED MICROBIOLOGY AND BIOTECHNOLOGY, SPRINGER, BERLIN, DE, vol. 76, no. 6, 16 August 2007 (2007-08-16), pages 1245-1253, XP019538784, ISSN: 1432-0614, DOI: 10.1007/S00253-007-1130-6
• MORADI MASOUMEH ET AL: "Introducing a novel bacterium, Vibrio neocaledonicus sp., with the highest corrosion inhibition efficiency", ELECTROCHEMISTRY COMMUNICATIONS, ELSEVIER, AMSTERDAM, NL, vol. 51, 13 December 2014 (2014-12-13), pages 64-68, XP029191248, ISSN: 1388-2481, DOI: 10.1016/J.ELECOM.2014.12.007
• CHENG, SHA: 'Effect of Vibrio Natriegens on Metal Corrosion in Sea Water and Research on Anti-pitting Method' SCIENCE -ENGINEERING (A), CHINA DOCTORAL DISSERTATIONS FULL-TEXT DATABASE 15 November 2009, pages B022 - 13, XP008185858
• PEDERSEN, A.: 'A Screening Method for Bacterial Corrosion of Metals' J OURNAL OF MICROBIOLOGICAL METHODS vol. 8, no. 4, 31 August 1988, pages 191 - 198, XP023699441

EP 3 231 897 B1

- **None**

- **None**

## Description

### Technical Field

The present application relates to the use of *Vibrio neocaledonicus sp.* as a biological metal corrosion inhibitor and a method for metal corrosion inhibition using a biological metal corrosion inhibitor.

### Technical Background

Due to being soaked in a marine environment for a long term, many metal products used in a marine environment such as ports and oil platforms corroded, and the regular maintenance cost is very high. It is necessary to develop protection techniques and methods for extending the maintenance cycle. For most current protection techniques, substances that release toxic substances to the environment are used and the processing cost is high. For example, in order to achieve a stainless effect, organic compound coatings were used (Finkenstadt et al., Biotechnology Letters, 2011, 33:1093-1100), or expensive alloying elements were added into the steel (Marcus et al., Corrosion Science, 1988, 28:589-602; and Sugimoto et al., Corrosion Science, 1977, 17:425-445). The development of environmentally friendly alternative technologies has become the focus of attention.

Currently, the research on bio-based materials as anticorrosive agents have been continuously expanded, since bio-based materials are biodegradable, reproducible, insecticide-free and of low toxicity, (Tang et al., Colloids and Surfaces A, 2010, 369:101-105; and Felix et al., Journal of Food Engineering, 2014, 125:7-16). These materials are ideal alternatives to conventional products, and are defined as "biological product" (US Department of Agriculture, Federal Register, 2005, 70:1792-1812).

It has been reported that biological products made from bacteria and metabolites thereof may slow metal corrosion (Zarasvand et al., International Biodeterioriation & Biodegradegradation, 2014, 87:66-74). Pedersen et al. firstly reported bacteria that may slow corrosion (Pedersen et al., Journal of microbiological methods, 1988, 8:191-198). Subsequently, Jayaraman et al. studied the mechanism of corrosion inhibition, and held the opinion that the formation of biological films is an important cause of this phenomenon (Jayaraman et al., Applied Microbiology and Biotechnology, 1977, 47:62-68). The corrosion inhibition of several bacteria was measured (Pedersen et al., Biofouling, 1989, 1:313-322; Pedersen et al., Biofouling, 1991, 3:1-11; Zuo et al., Applied Microbiology and Biotechnology, 2007, 76:1245-1253; and Ornek et al. Applied Microbiology and Biotechnology, 2002, 58:651-657). Among them, the bacterium having the best effect, i.e., *Pseudomonas mendocina* KR1, may increase the corrosion inhibition by ten times (Jayaraman et al., Applied Microbiology and Biotechnology, 1997, 48:11-17).

Although it has been found in studies that some bacteria have the effect of corrosion inhibition, there is still no report about preparing bacteria into metal corrosion inhibitors and applying them for corrosion inhibition.

E. Chalkiadakis et al., "Partial characterization of an exopolysaccharide secreted by a marine bacterium, Vibrio neocaledonicus sp. nov., from New Caledonia", JOURNAL OF APPLIED MICROBIOLOGY., GB, (20130412), vol. 114, no. 6, pages 1702 - 1712, describes a bacterial isolate belonging to a Vibrio species with metal binding capacity.

WO 02/40746 A2 and WO 2014/087139 A2 relate to methods for reducing corrosion of metal substrates with bacteria.

### Summary of the Invention

According to one aspect of the present application, the use of *Vibrio neocaledonicus sp.* as a biological metal corrosion inhibitor is provided, to inhibit the corrosion of metal materials under marine environments, salt solution conditions, and water environments by forming a biological film on the metal surface. The corrosion inhibitor may increase the corrosion resistance of a metal, and for carbon steels, the corrosion resistance may be increased by about 60 times.

The biological metal corrosion inhibitor contains *Vibrio neocaledonicus sp.*, wherein the *Vibrio neocaledonicus sp.*, is a *Vibrio neocaledonicus* KJ841877 strain with an accession number of MCCC 1K00266, and *Vibrio neocaledonicus sp.* is the active ingredient of the biological metal corrosion inhibitor.

A person skilled in the art will be able to select the content of living *Vibrio neocaledonicus sp.* in the biological metal corrosion inhibitor, according to actual needs and using conditions. Theoretically, the technical solution of the present application may be achieved as long as there is living *Vibrio neocaledonicus sp.* in the biological metal corrosion inhibitor. Furthermore, after the removal of *Vibrio neocaledonicus sp.,* the extracellular products thereof still have an inhibitive effect.

Preferably, the concentration of living *Vibrio neocaledonicus sp.* in the biological metal corrosion inhibitor is not less than 10,000 CFU/g. Further preferably, the concentration of living *Vibrio neocaledonicus sp.* in the biological metal corrosion inhibitor is not less than $10^5$ CFU/g. Further preferably, the concentration of living *Vibrio neocaledonicus sp.* in the biological metal corrosion inhibitor is not less than $5 \times 10^7$ CFU/g. Further preferably, the concentration of living *Vibrio neocaledonicus sp.* in the biological metal corrosion inhibitor is not less than $10^9$ CFU/g. Further preferably, the concentration of living *Vibrio neocaledonicus sp.* in the biological metal corrosion inhibitor is in a range from $10^9$ CFU/g to $10^{12}$ CFU/g.

The *Vibrio neocaledonicus sp.* is a *Vibrio neocaledonicus* KJ841877 strain.

Preferably, the method for preparing biological metal corrosion inhibitor includes inoculating *Vibrio neocaledonicus sp.* in a culture medium, and then performing a shaking fermentation culture for a time range from 12 hours to 48 hours at a temperature range from 20°C to 35°C and a rotation speed range from 100 r/min to 200 r/min, to obtain a biological metal corrosion inhibitor with a living bacterium concentration of no less than $10^5$ CFU/g. The culture medium may be a liquid-phase culture medium or a solid-phase culture medium. A person skilled in the art may select a suitable culture medium according to actual needs and using conditions.

According to another aspect of the present application, a method of using the biological metal corrosion inhibitor for metal corrosion inhibition is provided, wherein a metal is brought into contact with a system containing the biological thermal corrosion inhibitor, and the *Vibrio neocaledonicus sp.* grows on the surface of the metal to form a biological film with a metal corrosion inhibition effect, wherein the system containing the biological metal corrosion inhibitor contains *Vibrio neocaledonicus sp.* and a culture medium.

Preferably, the metal is contacted with the system containing the biological metal corrosion inhibitor in a way which is at least one selected from the group consisting of coating, spraying or immersion.

Preferably, the system containing the biological metal corrosion inhibitor is composed of *Vibrio neocaledonicus sp.* and a culture medium.

According to a preferred embodiment, the method of using the biological metal corrosion inhibitor for metal corrosion inhibition at least contains the following steps:

(A) cleaning the surface of a metal sample;

(B) coating a culture medium on the surface of the metal sample;

(C) coating or spraying the biological metal corrosion inhibitor onto the culture medium on the surface of the metal sample; wherein the mass ratio of the living *Vibrio neocaledonicus sp.* in the biological metal corrosion inhibitor to the culture medium is in a range from 0.01:1 to 10: 1; and

(D) placing the sample obtained in step (c) at a temperature range from 25 to 35 °C, and allowing the *Vibrio neocaledonicus sp.* growing on the surface of the metal for no less than 24 hours, so as to form a biological film with a corrosion inhibition effect.

A person skilled in the art may select an appropriate system and ratio of the *Vibrio neocaledonicus sp.* to the culture medium according to actual needs and using conditions.

Preferably, the system containing the biological metal corrosion inhibitor contains at least one selected from the group consisting of seawater, fresh water, mud, crude oil, and natural gas with water vapor. Further preferably, the system containing the metal corrosion inhibitor is seawater containing *Vibrio neocaledonicus sp.* and a culture medium and/or fresh water containing *Vibrio neocaledonicus sp.* and a culture medium. A person skilled in the art may select the pH range of the system containing the biological metal corrosion inhibitor according to actual needs and using conditions. In general, any pH would be appropriate as long as the *Vibrio neocaledonicus sp.* may survive.

Preferably, the pH of the system containing the biological metal corrosion inhibitor is in a range from 6 to 8.

Preferably, the temperature at which the *Vibrio neocaledonicus sp.* grows on the surface of a metal is in a range from 10°C to 35°C. Further preferably, the temperature at which the *Vibrio neocaledonicus sp.* grows on the surface of a metal is in a range from 25°C to 35°C.

According to a preferred embodiment, the method of using the biological metal corrosion inhibitor for metal corrosion inhibition at least contains the following steps:

(a) adding a culture medium into seawater and/or fresh water to obtain a mixed solution with a culture medium concentration from 0.1 g/mL to 20 g/mL;

(b) adding the biological metal corrosion inhibitor to the mixed solution obtained in step (a) to obtain a system containing the biological metal corrosion inhibitor, in which the concentration of the living *Vibrio neocaledonicus sp.* is in a range from $10^3$ CFU/mL to $10^{12}$ CFU/mL;

(c) cleaning the surface of a metal sample; and

(d) placing the cleaned metal sample obtained in step (c) into the system containing the biological metal corrosion inhibitor obtained in step (b) at a temperature range from 25°C to 35°C and allowing the *Vibrio neocaledonicus sp.* grow on the surface of the metal for no less than 24 hours, so as to form a biological film with a corrosion inhibition effect.

By a biological film formed by bacteria on the surface of a metal, the biological metal corrosion inhibitor is used to slow down and inhibit the corrosion of the metal, thereby achieving a protective purpose. Such biological film can be used as a corrosion inhibition layer to reduce corrosion on the surface of materials. This biological film may exist stably and its performance will increase over time. The main components of the biological film are organic substances such as protein and carbohydrates.

According to another aspect of the present disclosure, a metal material is provided whose surface is with a biological film with a metal corrosion inhibition effect; wherein the biological film is formed on the surface of the metal by any of the above-mentioned biological metal corrosion inhibitor and/or by the biological metal corrosion obtained using any of the above-mentioned methods.

Preferably, the metal is at least one selected from the group consisting of Fe-based metals, Cu-based metals, Al-based metals and alloys which are formed by at least two selected from Fe-based metals, Cu-based metals, Al-based metals. Further preferably, the metal is at least one selected from the group consisting of carbon steels, copper, copper alloys, aluminum, and aluminum alloys.

According to another aspect of the present application, the use of the metal material is provided. The metal material can be used in the environments of marine, lake, river or oil field. The metal material can be used as a metal material for hulls, pools, water tanks, water tanks for condensing towers, ship's ballast tanks, transportation pipelines for liquid and gas, and tanks.

The advantages of the present application include:

(1) The present application provides a novel biological metal corrosion inhibitor, which has an equivalent carbon steel protection effect compared with conventional plating or chemical nickel-plating methods. And the biological metal corrosion inhibitor is non-toxic, environmentally friendly, pollution free, reproducible and so on.

(2) The biological metal corrosion inhibitor provided in the present application can effectively suppress at least 98% corrosion of carbon steel, that is, the corrosion rate is reduced by more than 50 times. With increasing of the soaking time in seawater, the corrosion resistance will be improved. For example, the corrosion rate is reduced by 40 times after 24 hours soaking, and reduced by 60 times after 1 month soaking. The corrosion inhibition effect will always exist as long as maintaining the survival of bacteria.

(3) The biological metal corrosion inhibitor provided in the present application makes it possible that the ballast tank and metal parts which currently only can be used in fresh water are able to be used directly in seawater.

(4) The method for metal corrosion inhibition using the biological metal corrosion inhibitor is simple, and can greatly reduce the production and maintenance cost.

**Description of the Figures**

Figure 1 shows morphology of the carbon steel of Example 4 before the seawater immersion test.

Figure 2 shows morphology of the carbon steel of Example 4 after 1 day immersion in seawater without the biological metal corrosion inhibitor.

Figure 3 shows morphology of the carbon steel of Example 4 after 7 days soaking in seawater without the biological metal corrosion inhibitor.

Figure 4 shows morphology of the carbon steel of Example 4 after 14 days soaking in seawater without the biological metal corrosion inhibitor.

Figure 5 shows morphology of the carbon steel of Example 4 after 30 days soaking in seawater without the biological metal corrosion inhibitor.

Figure 6 shows morphology of the carbon steel of Example 4 after the removal of corrosive substances on the surface, before this step the carbon steel is soaked in seawater for 30 days without the biological metal corrosion inhibitor.

Figure 7 shows morphology of the carbon steel of Example 4 after 1 day immersion in seawater containing the biological metal corrosion inhibitor, J2.

Figure 8 shows morphology of the carbon steel of Example 4 after 7 days soaking in seawater containing the biological metal corrosion inhibitor, J2.

Figure 9 shows morphology of the carbon steel of Example 4 after 14 days soaking in seawater containing the biological metal corrosion inhibitor, J2.

Figure 10 shows morphology of the carbon steel of Example 4 after 30 days soaking in seawater containing the biological metal corrosion inhibitor, J2.

Figure 11 shows morphology of the carbon steel of Example 4 after the removal of corrosive substances on the surface, before this step the carbon steel is soaked in seawater for 30 days containing the biological metal corrosion inhibitor, J2.

Figure 12 shows Fourier transform infrared spectra of surfaces of M1 and M2 in Example 6.

## Detailed Description of the Embodiment

The present application will be further described by combining with Examples. It should be understood that these Examples are only used to illustrate the present application and not to limit the scope of the present application.
The *Vibrio neocaledonicus* KJ841877 strain, which was used in the examples, was purchased from the Marine Culture Collection of China (Address: No. 178, University Road, Xiamen City) with an accession number of 1K00266. The strain was kept in a broth medium for marine bacteria containing 30% of glycerol by weight at -80°C.
In the Examples, the chemical composition of the samples was analyzed by Fourier transform infrared spectroscopy (FT-IR) and X-ray photoelectron spectroscopy (XPS).
In the Examples, the sterile seawater is a sterile solution simulating the composition of seawater with the composition of: 23.476g/L NaCl; 1.469 g/L $CaCl_2 \cdot 6H_2O$; 0.192 g/L $NaHCO_3$; 10.61 g/L $MgCl_2 \cdot 6H_2O$; 3.917 g/L $Na_2SO_4$; 0.026 g/L boric acid; 0.667 g/L KCl; and 0.096 g/L KBr.

**Example 1** Preparation of the biological metal corrosion inhibitors

The purchased strain was stored in a broth medium for marine bacteria containing 30wt% of glycerol at -80°C. 100 μL of the above-mentioned broth medium containing the strain was inoculated into solid LB medium which was placed in a culture dish with a diameter of 10 cm, and then being fermented and cultured in an incubator at 30°C for 24 hours, to obtain the biological metal corrosion inhibitor which was denoted as J1.
100 μL of the above-mentioned broth medium containing the strain was inoculated into 100 cc of liquid LB medium, and then being shaking fermented and cultured at 30°C and a rotation speed of 130r/min for 24 hours, to obtain the biological metal corrosion inhibitor which was denoted as J2.
100 μL of the above-mentioned broth medium containing the strain was inoculated into 100 cc of liquid medium (artificial seawater + 3 g/L peptone + 1.5 g/L yeast extract), and then being shaking fermented and cultured at 30°C and a rotation speed of 130r/min for 24 hours, to obtain the biological metal corrosion inhibitor which was denoted as J3.

**Example 2** Determination of living bacteria concentration in the biological metal corrosion inhibitors

About 1 $mm^3$ of the biological metal corrosion inhibitor J1 prepared in Example 1 was diluted in 1L of sterilized seawater. 0.1 mL of the diluted solution was added onto a plate, and then being coated on the whole surface of the plate with a sterile stick, and then being cultured at 30°C for 24 hours. Subsequently, the number of colonies was calculated to obtain the living bacteria concentration in J1, i.e., $8 \times 10^9$ CFU/g.
0.1 g of the biological metal corrosion inhibitor J2 and J3 was diluted in 1L of sterilized seawater, respectively. 0.1 mL of the diluted solution was added onto a plate, and then being coated on the whole surface of the plate with a sterile stick, and then being cultured at 30°C for 24 hours. Subsequently, the number of colonies was calculated to obtain the living bacteria concentration in J2 and J3, i.e., $8 \times 10^9$ CFU/g and $5 \times 10^7$ CFU/g, respectively.

**Example 3** Determination of corrosion inhibition property of A36 carbon steel

A36 carbon steel was used as a metal sample. The metal sample was cut into a square with a length of 10 cm, and a copper wire was welded to the back, and then the back side was encapsulated with epoxy resin. The front of the metal sample was flattened with 2000 mesh sandpaper; and after being washed in 95% ethanol and deionized water, the metal sample was immersed in 95% ethanol and sterilized under UV light for 1 hour.
Experimental sample group and control sample group respectively used two groups of the obtained metal sample, and each group contained 4 metal samples.
Experimental sample group: the metal samples were respectively placed in a flask filled with sterile seawater, adding peptone to make the concentration of peptone in the sterile seawater to be 3 g/L; and then the biological metal corrosion inhibitor J1 obtained in Example 1 was added to make the living bacteria concentration in the obtained system to be $10^5$ CFU/g; and then the flask was placed in an incubator shaker at a temperature of 30°C and a rotation speed of 80 rpm/min.

Control sample group: the metal samples were respectively placed in a flask filled with sterile seawater, and the flask was placed in an incubator shaker at a temperature of 30°C and a rotation speed of 80 rpm/min.

After being treated for 1 day, 7 days, 14 days and 30 days, the samples of the experimental sample group and the control sample group were picked up, respectively. The corrosion rate of the samples was evaluated using AC impedance and polarization curve.

The method and steps for measuring AC impedance and polarization curve were as follows:

A carbon steel sheet with a size of 10 mm × 10 mm × 3 mm was used as a working electrode. The AC impedance and the polarization curve were measured using an AutoLabab302 electrochemical workstation (PGSTAT302, Metrohm AG). For electrochemical tests, three-electrode system was used, in which the working electrode was a test sample with an area of 10 mm × 10mm, and the counter electrode was a platinum electrode, and the reference electrode was a saturated calomel electrode. The test medium was simulated seawater. For EIS (Electrochemical Impedance Spectroscopy), the test frequency range was from 10kHz to 5Hz, and the test excitation potential was 10m V The potential scan rate of the polarization curve was 0.166 mV/s.

The corrosion rate is represented by the corrosion current density, which was calculated using the formula below:

$$Corrosion\ rate = \frac{\int_{0}^{365*24*3600} idt}{nF} \cdot m \cdot 10^6 \left( \mu m / Y \right)$$

Wherein, $i$ represents the corrosion current density (unit: ampere/square meter); $dt$ represents the time differential (unit: seconds); F represents the Faraday constant; $n$ represents the change of the metal valence state; $m$ represents the molar volume of the metal (unit: cubic meter/mole), which equals to the atomic weight/density; and ($\mu m/Y$) represents the unit of corrosion rate (microns/year).

The corrosion electric resistance was measured by AC impedance.

The corrosion inhibition effect is represented by corrosion inhibition ratio P (%), which was calculated using the following formula:

$$P(\%) = \frac{R_{ct} - R_{ct,0}}{R_{ct}} \times 100$$

Wherein, $R_{ct,0}$ represents the charge transfer resistance of the blank solution; and $R_{ct}$ represents the charge transfer resistance of the bacteria containing solution.

The corrosion rate, corrosion electric resistance and corrosion inhibition ratio data were shown in Table 1. It can be seen from the data in Table 1 that the biological metal corrosion inhibitor provided in the present application could effectively suppress at least 98% of carbon steel corrosion, that is, the corrosion rate was reduced by more than 50 times. With the increase of the soaking time, the corrosion resistance was further improved. For example, the corrosion rate was reduced by 40 times after being soaked for 24 hours, and reduced by 60 times after being soaked for 1 month soaking. The corrosion inhibition effect will always exist as long as maintaining the survival of bacteria.

Table 1

| Composition | Treatment Time | Corrosion rate ($\mu$m/year) | Corrosion electric resistance (ohm·cm$^2$) | Corrosion inhibition ratio (%) |
|---|---|---|---|---|
| Control sample | Initial state | 63.75 | 830 | - |
| | 1 day | 66.5 | 1055 | 21.32 |
| | 7 days | 81.5 | 1753 | 52.65 |
| | 14 days | 86.5 | 1832 | 54.69 |
| | 30 days | 68.2 | 1311 | 36.67 |
| Sea water added with corrosion inhibitor | 1 days | 14.3 | 40700 | 97.96 |
| | 7 days | 11.4 | 42100 | 98.03 |
| | 14 days | 9.87 | 44800 | 98.15 |
| | 30 days | 8.25 | 49000 | 98.31 |

**Example 4** Determination of corrosion inhibition property for medium-carbon steel under a condition of circulating seawater

Medium-carbon steel was used as a metal sample. The metal sample was cut into a square with a length of 10 cm, and a copper wire was welded to the back, and then the back side was encapsulated with epoxy resin. The front of the metal sample was flattened with 2000 mesh sandpaper; after being washed in 95% ethanol and deionized water, the metal sample was immersed in 95% ethanol and sterilized under UV light for 1 hour.

Experimental sample group and control sample group respectively used two groups of the obtained metal sample, and each group contained 4 metal samples.

Experimental sample group: the metal samples were respectively placed in a sealed container filled with 30L of sterile seawater, adding peptone to make the concentration of peptone in the sterile seawater to be 3 g/L; and then the biological metal corrosion inhibitor J2 obtained in Example 1 was added to make the living bacteria concentration in the obtained system to be $10^9$ CFU/g; and then the temperature of the sealed container was maintained at 30°C, and a closed circulating pump was used for circulating water flow. Every 2 days, 50% of the liquid in the sealed container was replaced with an equal volume of sterile seawater.

Control sample group: the metal samples were respectively placed in a sealed container filled with 30L of sterile seawater. The temperature of the sealed container was maintained at 30°C and a closed circulating pump was used for circulating water flow. Every 2 days, 50% of the liquid in the sealed container was replaced with an equal volume of sterile seawater.

After being treated for 1 day, 7 days, 14 days and 30 days, the samples of the experimental sample group and the control sample group were picked up, respectively. The electrochemical data of each sample were measured and the surface morphologies of each sample were recorded using a digital camera.

The method for measuring electrochemical data was as follows:

A carbon steel sheet test sample with a size of 100 mm × 100 mm was used as a working electrode. The corrosion current and potential were measured using an AutoLabab302 electrochemical workstation (PGSTAT302). For electrochemical tests, two electrodes were used; wherein the working electrode was the test sample and the counter electrode was a platinum electrode.

Results were shown in Table 2. It can be seen from experimental results that the electrode potential of the samples increased with the exposure time in seawater without the corrosion inhibitor, and in seawater containing the corrosion inhibitor the electrode potential remained stable. The shift towards positive direction of the electrode potential could make ferrite enter into the passive area, leading to the occurrence of surface pitting. In the seawater without metal corrosion inhibitor, the corrosion current firstly decreases, and then increases, indicating that initial corrosion products play a protective role, but corrosion intensify with the increase of time. However, in the seawater containing metal corrosion inhibitor, the corrosion current decreases to some level and then keep unchanged.

Table 2

| Composition | Treatment Time | Potential (mV vs Ag/AgCl) | Corrosion current density ($\mu$A/ 100cm$^2$) |
|---|---|---|---|
| Seawater without corrosion inhibitor | Initial state | -560 | 360 |
| | 1 day | -420 | 320 |
| | 7 days | -270 | 18 |
| | 14 days | -167 | 5 |
| | 30 days | -25 | 320 |
| Sea water added with corrosion inhibitor | 1 days | -650 | 32 |
| | 7 days | -520 | 17 |
| | 14 days | -670 | 5 |
| | 30 days | -530 | 1 |

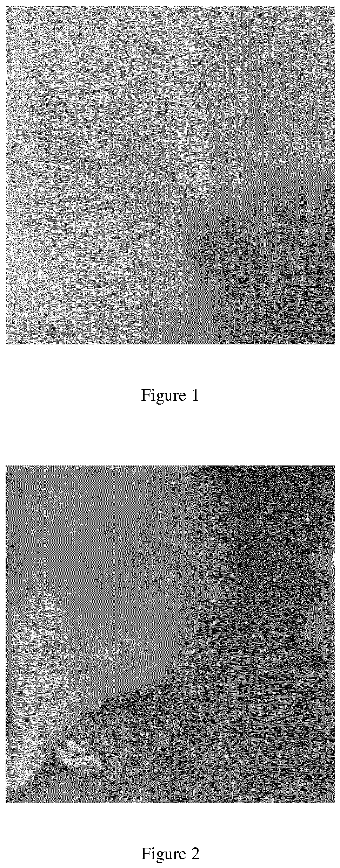

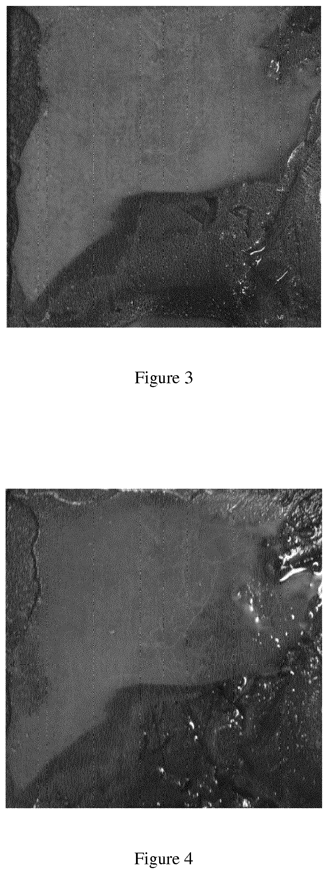

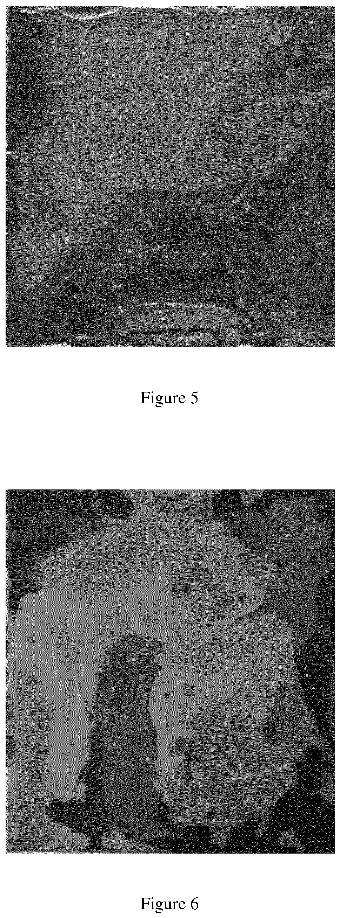

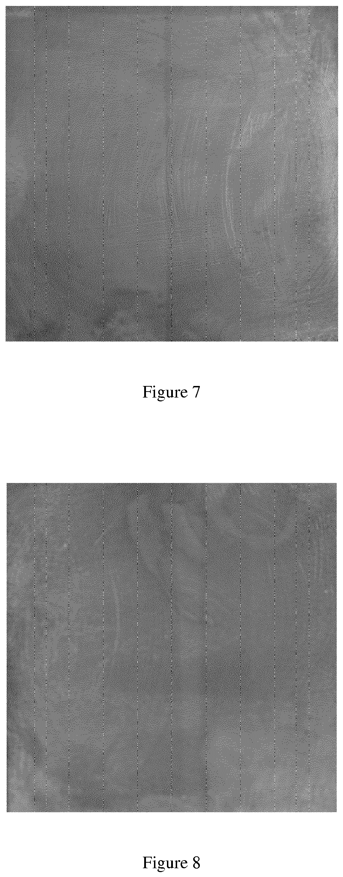

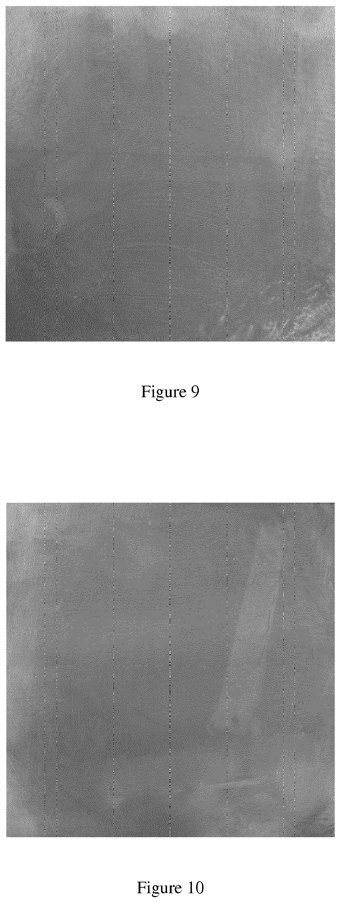

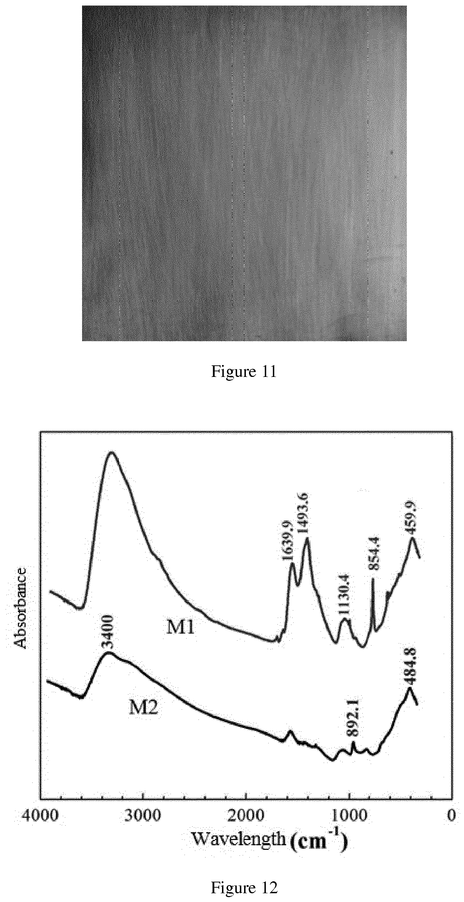

The surface topography of each sample was shown in Figures 1 to 10. It can be seen from the figures that: for soaking in the blank solution without bacteria, with the soaking time increased, a large area of red rust appeared on the steel base after the first day (seeing Figure 2); and after 7 days, the area of rust and the thickness of rust layer increased, and part of the rust layer was separated from the steel base; and after 14 and 30 days, the area and thickness of rust layer further increased (seeing Figures 4 and 5). The rust layer on the surface of the sample soaked for 30 days was removed by ultrasonic cleaning, and it was found that the steel base had serious corrosion. However, after being soaked for 30 days, the samples soaked in seawater containing the bacteria substantially had no rust (seeing Figures 7, 8, 9 and 10) and the morphology of the base (seeing Figure 11) was substantially the same as that of initial state (seeing Figure 1).

**Example 5**

The conditions and steps of Example 5 were the same as those in Example 3 except that the metal sample was replaced by copper and the biological metal corrosion inhibitor was replaced by J3.

The corrosion rate, corrosion electric resistance and corrosion inhibition ratio results were shown in Table 3. It can be seen from the data in Table 3 that the metal corrosion inhibitor of the present application also exhibited an excellent corrosion inhibition effect for pure copper metal.

Table 3

| Experimental condition | time | Corrosion rate ($\mu$m/year) | Corrosion electric resistance (ohm·cm$^2$) | Corrosion inhibition ratio (%) |
|---|---|---|---|---|
| Pure copper in seawater | Initial state | 61.23 | 475.73 | - |
| Pure copper in seawater without metal corrosion inhibitor | 7 days | 17.4 | 1495 | 68.18 |
| Pure copper in seawater containing metal corrosion inhibitor | | 11.02 | 85800 | 99.44 |
| Pure copper in seawater without metal corrosion inhibitor | 14 days | 22.5 | 2150.33 | 77.88 |
| Pure copper in seawater containing metal corrosion inhibitor | | 1.93 | 718300 | 99.93 |

**Example 6** Characterization of biological film on metal surface

A36 carbon steel was used as a metal sample. The metal sample was cut into a square with a length of 10 cm, and a copper wire was welded to the back, and then the back side was encapsulated with epoxy resin. The front of the metal sample was flattened with 2000 mesh sandpaper; after being washed in 95% ethanol and deionized water, the metal sample was immersed in 95% ethanol and sterilized under UV light for 1 hour.

Experimental sample group and control sample group respectively used two groups of the obtained metal sample, and each group contained 4 metal samples.

Experimental sample group: the metal samples were respectively placed in a flask filled with sterile seawater, peptone was added, and the concentration of peptone in the sterile seawater was 3 g/L; and then the biological metal corrosion inhibitor J1 obtained in Example 1 was added to make the living bacteria concentration in the obtained system to be $10^5$ CFU/g; and then the flask was placed in an incubator shaker at a temperature of 30°C and a rotation speed of 80 rpm/min for 10 days, to obtain a metal sample which was denoted as M1.

Control sample group: the metal samples were respectively placed in a flask filled with sterile seawater; and then the flask was placed in an incubator shaker at a temperature of 30°C and a rotation speed of 80 rpm/min for 10 days, to obtain a metal sample which was denoted as M2.

The surface chemical composition of M1 and M2 was analyzed by Fourier transform infrared spectroscopy (FT-IR) on a Nicolet 6700 (Thermo, USA) infrared spectrometer, respectively, and the results were shown in Figure 12.

It can be seen from Figure 12 that for the contrast sample in blank test without metal corrosion inhibitor, only two peaks of water and $\gamma$-$Fe_2O_3$ were observed on the surface of M2; and for the sample M1 soaked in a solution containing metal corrosion inhibitor, at least 6 peaks were observed. Wherein, the peaks of 1639.9 cm$^{-1}$ and 1493.6 cm$^{-1}$ corresponded to the C=O bond in carboxylic acid anion and amino acid (Ghafari et al., International Biodeterioration & Biodegradation, 2013, 80: 29-33). The amino acid should correspond to the protein in the biological film (Reubena et al., Water Research, 2014, 64: 123-133). Two other peaks at 1130.4 cm$^{-1}$ and 854.4 cm$^{-1}$ corresponded to the C-O bond in polysaccharides and lipids, respectively (Hessler et al., Water Research, 2012, 46: 4687-4696). Schmitt and Flemming (International Biodeterioration & Biodegradation, 1998, 41: 1-11) indicated that the peak from 1150cm$^{-1}$ to 750cm$^{-1}$ belonged to polysaccharide compounds. Therefore, Fourier transform infrared spectroscopy confirmed that the biological film contained polysaccharides and proteins.

**Claims**

1.  Use of *Vibrio neocaledonicus sp.* as a biological metal corrosion inhibitor, wherein the *Vibrio neocaledonicus sp.* is a *Vibrio neocaledonicus* KJ841877 strain with an accession number of MCCC 1K00266.

2.  The use according to claim 1, wherein the concentration of living *Vibrio neocaledonicus sp.* in the biological metal corrosion inhibitor is in a range from $10^9$ CFU/g to $10^{12}$ CFU/g.

3.  A method for metal corrosion inhibition using a biological metal corrosion inhibitor,
    wherein the biological metal corrosion inhibitor comprises *Vibrio neocaledonicus sp.,* which is a *Vibrio neocaledonicus* KJ841877 strain with an accession number of MCCC 1K00266,
    wherein a metal is brought into contact with a system containing the biological metal corrosion inhibitor, and the *Vibrio neocaledonicus sp.* grows on the surface of the metal to form a biological film with a metal corrosion inhibition effect, and
    wherein the system containing the biological metal corrosion inhibitor contains *Vibrio neocaledonicus sp.* and a culture medium.

4.  The method according to claim 3, wherein the concentration of living *Vibrio neocaledonicus sp.* in the biological metal corrosion inhibitor is in a range from $10^9$ CFU/g to $10^{12}$ CFU/g.

5.  The method according to claim 3, wherein the system containing the biological metal corrosion inhibitor contains at least one selected from the group consisting of seawater, fresh water, mud, crude oil, and natural gas with water vapor.

6.  The method according to claim 3, wherein the pH of the system containing the biological metal corrosion inhibitor is in a range from 5 to 9.

7.  The method according to claim 3, wherein the temperature at which the *Vibrio neocaledonicus sp.* grows on the surface of the metal is in a range from 10°C to 35°C.

8.  A metal material, whose surface is with a biological film with a metal corrosion inhibition effect; wherein the biological film is obtained using the method according to any of claims 3 to 7.

9.  The metal material according to claim 8, wherein the metal is at least one selected from the group consisting of carbon steels, copper, copper alloys, aluminum, and aluminum alloys.

10. Use of the metal material according to claim 8 in the environments of marine, lake, river, pool, water tank, condensing tower or oil field.

**Patentansprüche**

1. Verwendung von *Vibrio neocaledonicus sp.* als biologischer Metallkorrosionsinhibitor, wobei *Vibrio neocaledonicus sp.* ein *Vibrio neocaledonicus* KJ841877-Stamm mit der Hinterlegungsnummer MCCC 1K00266 ist.

2. Verwendung nach Anspruch 1, wobei die Konzentration von lebendem *Vibrio neocaledonicus sp.* in dem biologischen Metallkorrosionsinhibitor in einem Bereich von $10^9$ CFU/g bis $10^{12}$ CFU/g liegt.

3. Verfahren zur Metallkorrosionsinhibierung unter Verwendung eines biologischen Metallkorrosionsinhibitors, wobei der biologische Metallkorrosionsinhibitor *Vibrio neocaledonicus sp.* umfasst, bei dem es sich um einen *Vibrio neocaledonicus* KJ841877-Stamm mit einer Hinterlegungsnummer MCCC 1K00266 handelt, wobei ein Metall in Kontakt mit einem System gebracht wird, das den biologischen Metallkorrosionsinhibitor enthält, und *Vibrio neocaledonicus sp.* auf der Oberfläche des Metalls wächst, um einen biologischen Film mit einer Metallkorrosionsinhibitionswirkung zu bilden, und wobei das System, das den biologischen Metallkorrosionsinhibitor enthält, *Vibrio neocaledonicus sp.* und ein Kulturmedium enthält.

4. Verfahren nach Anspruch 3, wobei die Konzentration von lebendem *Vibrio neocaledonicus sp.* in dem biologischen Metallkorrosionsinhibitor in einem Bereich von $10^9$ CFU/g bis $10^{12}$ CFU/g liegt.

5. Verfahren nach Anspruch 3, wobei das System, das den biologischen Metallkorrosionsinhibitor enthält, mindestens eines enthält, das aus der Gruppe ausgewählt ist, die aus Seewasser, Süßwasser, Schlamm, Rohöl und Erdgas mit Wasserdampf besteht.

6. Verfahren nach Anspruch 3, wobei der pH-Wert des Systems, das den biologischen Metallkorrosionsinhibitor enthält, in einem Bereich von 5 bis 9 liegt.

7. Verfahren nach Anspruch 3, wobei die Temperatur, bei der *Vibrio neocaledonicus sp.* auf der Oberfläche des Metalls wächst, in einem Bereich von 10°C bis 35°C liegt.

8. Metallmaterial, dessen Oberfläche mit einem biologischen Film mit einer Metallkorrosionsinhibitionswirkung versehen ist; wobei der biologische Film unter Verwendung des Verfahrens nach einem der Ansprüche 3 bis 7 erhalten wird.

9. Metallmaterial nach Anspruch 8, wobei das Metall mindestens eines ist, ausgewählt aus der Gruppe bestehend aus Kohlenstoffstählen, Kupfer, Kupferlegierungen, Aluminium und Aluminiumlegierungen.

10. Verwendung des Metallmaterials nach Anspruch 8 in den Umgebungen von Meer, See, Fluss, Pool, Wassertank, Kondensationsturm oder Ölfeld.

**Revendications**

1. Utilisation de *Vibrio neocaledonicus sp.* comme inhibiteur biologique de la corrosion des métaux, dans laquelle *Vibrio neocaledonicus sp.* est une souche *Vibrio neocaledonicus* KJ841877 avec un numéro d'accès de MCCC 1K00266.

2. Utilisation selon la revendication 1, la concentration de *Vibrio neocaledonicus sp.* vivant dans l'inhibiteur de corrosion métallique biologique étant dans une gamme de $10^9$ CFU/g à $10^{12}$ CFU/g.

3. Procédé d'inhibition de la corrosion des métaux utilisant un inhibiteur biologique de corrosion des métaux, dans lequel l'inhibiteur biologique de corrosion des métaux comprend l'espèce *Vibrio neocaledonicus,* qui est une souche *Vibrio neocaledonicus* KJ841877 avec un numéro d'accès de MCCC 1K00266,

dans lequel un métal est mis en contact avec un système contenant l'inhibiteur biologique de corrosion des métaux, et la *Vibrio neocaledonicus sp.* se développe sur la surface du métal pour former un film biologique avec un effet d'inhibition de la corrosion des métaux, et

dans lequel le système contenant l'inhibiteur biologique de corrosion des métaux contient l'espèce *Vibrio neocaledonicus* et un milieu de culture.

4. Procédé selon la revendication 3, dans lequel la concentration de *Vibrio neocaledonicus sp.* vivant dans l'inhibiteur biologique de corrosion des métaux est dans une gamme de $10^9$ CFU/g à $10^{12}$ CFU/g.

5. Procédé selon la revendication 3, dans lequel le système contenant l'inhibiteur biologique de corrosion des métaux contient au moins un élément choisi dans le groupe constitué par l'eau de mer, l'eau douce, la boue, le pétrole brut et le gaz naturel avec de la vapeur d'eau.

6. Procédé selon la revendication 3, dans lequel le pH du système contenant l'inhibiteur biologique de corrosion des métaux est dans une gamme de 5 à 9.

7. Procédé selon la revendication 3, dans lequel la température à laquelle le *Vibrio neocaledonicus sp.* se développe à la surface du métal est dans une gamme de 10°C à 35°C.

8. Matériau métallique, dont la surface est pourvu d'un film biologique avec un effet d'inhibition de la corrosion du métal ; dans lequel le film biologique est obtenu en utilisant la méthode selon l'une quelconque des revendications 3 à 7.

9. Matériau métallique selon la revendication 8, dans lequel le métal est au moins un métal choisi dans le groupe constitué par les aciers au carbone, le cuivre, les alliages de cuivre, l'aluminium et les alliages d'aluminium.

10. Utilisation du matériau métallique selon la revendication 8 dans les environnements marines et ceux de lac, rivière, piscine, réservoir d'eau, tour de condensation ou champ pétrolifère.

Figure 1

Figure 2

Figure 3

Figure 4

Figure 5

Figure 6

Figure 7

Figure 8

Figure 9

Figure 10

Figure 11

Figure 12

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0240746 A2
- WO 2014087139 A2

**Non-patent literature cited in the description**

- **FINKENSTADT et al.** *Biotechnology Letters,* 2011, vol. 33, 1093-1100
- **MARCUS et al.** *Corrosion Science,* 1988, vol. 28, 589-602
- **SUGIMOTO et al.** *Corrosion Science,* 1977, vol. 17, 425-445
- **TANG et al.** *Colloids and Surfaces A,* 2010, vol. 369, 101-105
- **FELIX et al.** *Journal of Food Engineering,* 2014, vol. 125, 7-16
- Federal Register. US Department of Agriculture, 2005, vol. 70, 1792-1812
- **ZARASVAND et al.** *International Biodeterioriation & Biodegradegradation,* 2014, vol. 87, 66-74
- **PEDERSEN et al.** *Journal of microbiological methods,* 1988, vol. 8, 191-198
- **JAYARAMAN et al.** *Applied Microbiology and Biotechnology,* 1977, vol. 47, 62-68
- **PEDERSEN et al.** *Biofouling,* 1989, vol. 1, 313-322
- **PEDERSEN et al.** *Biofouling,* 1991, vol. 3, 1-11
- **ZUO et al.** *Applied Microbiology and Biotechnology,* 2007, vol. 76, 1245-1253
- **ORNEK et al.** *Applied Microbiology and Biotechnology,* 2002, vol. 58, 651-657
- **JAYARAMAN et al.** *Applied Microbiology and Biotechnology,* 1997, vol. 48, 11-17
- **E. CHALKIADAKIS et al.** Partial characterization of an exopolysaccharide secreted by a marine bacterium, Vibrio neocaledonicus sp. nov., from New Caledonia. *JOURNAL OF APPLIED MICROBIOLOGY,* 12 April 2013, vol. 114 (6), 1702-1712
- **GHAFARI et al.** *International Biodeterioration & Biodegradation,* 2013, vol. 80, 29-33
- **REUBENA et al.** *Water Research,* 2014, vol. 64, 123-133
- **HESSLER et al.** *Water Research,* 2012, vol. 46, 4687-4696
- **SCHMITT ; FLEMMING.** *International Biodeterioration & Biodegradation,* 1998, vol. 41, 1-11